Europäisches Patentamt

European Patent Office

Office européen des brevets

(19)

(11) Publication number: **0 623 308 A1**

## EUROPEAN PATENT APPLICATION

(12)

(21) Application number: **94107137.5**

(22) Date of filing: **06.05.94**

(51) Int. Cl.5: **A61B 5/00**

(30) Priority: **07.05.93 US 59161**

(43) Date of publication of application:
**09.11.94 Bulletin 94/45**

(84) Designated Contracting States:
**AT BE CH DE DK ES FR GB GR IE IT LI LU MC NL PT SE**

(71) Applicant: **DIASENSE, INC.**
**The Bourse,**
**Bldg. 2500, 2nd Floor,**
**2275 Swallow Hill Road**
**Pittsburgh, Pennsylvania 15220 (US)**

(72) Inventor: **Wiggins, Richard L.**
**400 Nehrig Drive**
**Indiana, Pennsylvania 15701 (US)**

(74) Representative: **Patentanwälte Beetz - Timpe -**
**Siegfried Schmitt-Fumian - Mayr**
**Steinsdorfstrasse 10**
**D-80538 München (DE)**

(54) Method for non-invasive measurement of concentration of constituents in blood.

(57) A method for the detection of the concentration of an analyte in the blood of a living animal or person includes the steps of non-invasively irradiating a body part of the animal or person having a first blood volume to tissue volume ratio and detecting radiation emitted from the body part, non-invasively irradiating a body part having a second blood volume to tissue volume ratio and detecting radiation emitted from the body part, and calculating the concentration of the blood analyte based on the detected emitted radiation.

FIG. 1

Background of the Invention.

This invention relates to techniques for non-invasively detecting the concentration of analytes in the blood of living animals or persons, and in particular to the use of infrared and near-infrared spectroscopic techniques for the non-invasive detection of glucose concentrations in the blood of humans.

In the diagnosis and treatment of various conditions, it is important to measure the concentration of various constituents in the blood. For example, in the treatment of diabetes, the concentration of glucose in the blood must be measured on a periodic basis. For persons experiencing insulin-dependent or Type I diabetes, it is often necessary or desirable to measure blood glucose concentration several times each day. Obtaining accurate readings of cholesterol concentrations is important in the prevention of coronary artery disease. The measurement of the concentration of other blood analytes such as bilirubin and alcohol, is also important for various diagnostic purposes.

The accurate measurement of concentrations of such blood constituents, as it is now practiced, requires obtaining a blood sample, such as by pricking a finger. The obtaining of blood samples by invasive techniques, such as pricking the finger, is both painful and inconvenient. In the case of diabetics, the need to lance a finger several times a day to monitor glucose levels results in a build-up of substantial scar tissue. Indeed, many diabetics are believed not to monitor their glucose level as frequently as recommended because of the pain and inconvenience of the invasive method. The result of such a failure to monitor glucose levels is a greater risk of experiencing the long-term health effects of diabetes. These health effects include damage to the eyes, resulting in partial and often total loss of vision, and damage to the extremities, which can result in the need to amputate. Millions of individuals in the United States alone suffer from diabetes. As a result, the failure of individuals afflicted with diabetes reliably to monitor their glucose levels is a significant public health problem.

In order to provide an alternative to the existing invasive blood glucose monitoring techniques, various non-invasive blood glucose detection techniques have been proposed. One of the most promising of these techniques is the non-invasive infrared spectroscopic technique. In this technique, a portion of the patient's skin is irradiated with infrared or near-infrared radiation, either at selected wavelengths or over a portion of the spectrum. Radiation that is either back-scattered or transmitted through a body part such as the finger, is then measured. By appropriate spectroscopic analysis techniques, it has been hoped that the concentration of glucose in the blood can be determined.

However, no instrument using the non-invasive infrared, or near infrared, technique, has achieved accuracy sufficient to match that of existing invasive techniques. As a result, no instrument using the non-invasive infrared or near-infrared technique has achieved accuracy sufficient to be approved by regulators for use in the United States.

It is accordingly an object of this invention to provide an improvement in the technique for the non-invasive detection of analytes in the bloodstream of living animals or persons.

It is a particular object of this invention to provide an improvement in techniques for the detection of concentrations of glucose in the bloodstream of humans suffering from diabetes.

Further objects and advantages of this invention will become apparent from the detailed description of a preferred embodiment which follows.

Summary of the Invention.

A method for the detection of the concentration of an analyte in blood of a living animal or person comprises the steps of non-invasively irradiating a body part of the animal or person having a first blood volume to tissue volume ratio, detecting radiation emitted from the body part, non-invasively irradiating a body part having a second blood volume to tissue volume ratio, detecting radiation emitted from the body part, and calculating the concentration of the analyte in blood based on the detected radiation.

A method for calibrating an instrument for non-invasively determining the concentration of an analyte in the blood of a living animal or person comprises the steps of non-invasively irradiating a body part of the animal or person having a first blood volume to tissue volume ratio and detecting radiation emitted from the body part to obtain a first absorbance spectrum, non-invasively irradiating a body part of the animal or person having a second blood volume to tissue volume ratio, and detecting the radiation emitted by the body part, to obtain a second absorbance spectrum, invasively detecting the concentration of the analyte in the blood of the animal substantially simultaneously with said steps of irradiating to obtain the first and second absorbance spectra, and employing the first absorbance spectrum, the second absorbance spectrum, and the invasively-detected concentration, to calibrate the instrument.

An apparatus for the non-invasive detection of the concentration of a constituent of blood of a living animal or person includes means for non-invasively irradiating a body part of the animal or person, means for detecting radiation emitted from the body part, and data processing means for receiving a first signal from the detecting means representative of the intensity of radiation detected during irradiation of a body part having a first blood volume to tissue volume ratio and calculating and storing a first absorbance spectrum based on the first signal; receiving a second signal from the detecting means representative of the intensity of radiation detected during irradiation of a body part having a second blood volume to tissue volume ratio and calculating and storing a second absorbance spectrum based on the second signal; and calculating from the first absorbance spectrum and the second absorbance spectrum the concentration of the constituent in the blood.

An apparatus for calibrating an instrument for non-invasive determination of the concentration of an analyte in the blood of a living animal or person includes means for non-invasively irradiating a body part of the animal or person; means for detecting radiation emitted from the body part; and data processing means for receiving a first signal from the detecting means representative of the intensity of radiation detected during irradiation of a body part having a first blond volume to tissue volume ratio and calculating and storing a first absorbance spectrum based on the first signal, receiving a second signal from the detecting means representative of the intensity of radiation detected during irradiation of a body part having a second blood volume to tissue volume ratio and calculating and storing a second absorbance spectrum based on the second signal; receiving an input representing an invasively-detected concentration of the analyte in the blood of the animal; and based on the first absorbance spectrum, the second absorbance spectrum and the invasively-detected concentration, calibrating the instrument.

Brief Description of the Figures.

Figure 1 is a sectional, schematic representation of a step in a method of non-invasive detection of analyte concentration in the blood.

Figure 2 is a schematic representation of a method of non-invasive detection of analyte concentration in the blood stream.

Figure 3 is a sectional, schematic representation a step in non-invasive detection of analyte concentration in the blood.

Figure 4 is a flow diagram illustrating the steps in a method for the calibration of an apparatus for the non-invasive detection of analyte concentration in the blood.

Figure 5 is a flow diagram illustrating the steps in a method for the non-invasive detection of analyte concentration in the blood.

Figures 6A and 6B illustrate the step of irradiating an earlobe at two different blood volume to tissue volume ratios in a method according to the invention, and Figures 6C and 6D illustrate the step of irradiating an earlobe and detecting radiation transmitted through the earlobe at two different blood volume to tissue volume ratios in a method according to the invention.

Figure 7A and 7B illustrate the step of irradiating a finger tip at two different blood volume to tissue volume ratios in a method of the invention.

Figures 8A and 8B illustrate the step of irradiating the skin over a vein at two different blood volume to tissue volume ratios in a method of the invention.

Figure 9 is a block diagram illustrating an apparatus for obtaining two different spectra showing absorbance at a first blood volume to tissue volume ratio and at a second blood volume to tissue volume ratio in a method of the invention.

Figure 10A is a schematic representation of an apparatus for obtaining a first absorption spectrum at a first blood volume to tissue volume ration and a second absorption spectrum at a second blood volume to tissue volume ratio in a method of the invention.

Figure 10B is a flow diagram illustrating the steps in a method of calibrating an apparatus for the non-invasive detection of blood analyte concentrations according to the invention.

Figure 10C is a flow diagram illustrating the steps in a method of obtaining a blood analyte concentration reading in accordance with the invention.

Figures 11A and 11B illustrate the step of irradiating an inner surface of the mouth at two different blood volume to tissue volume ratios in a method in accordance with the invention.

Detailed Description of a Preferred Embodiment.

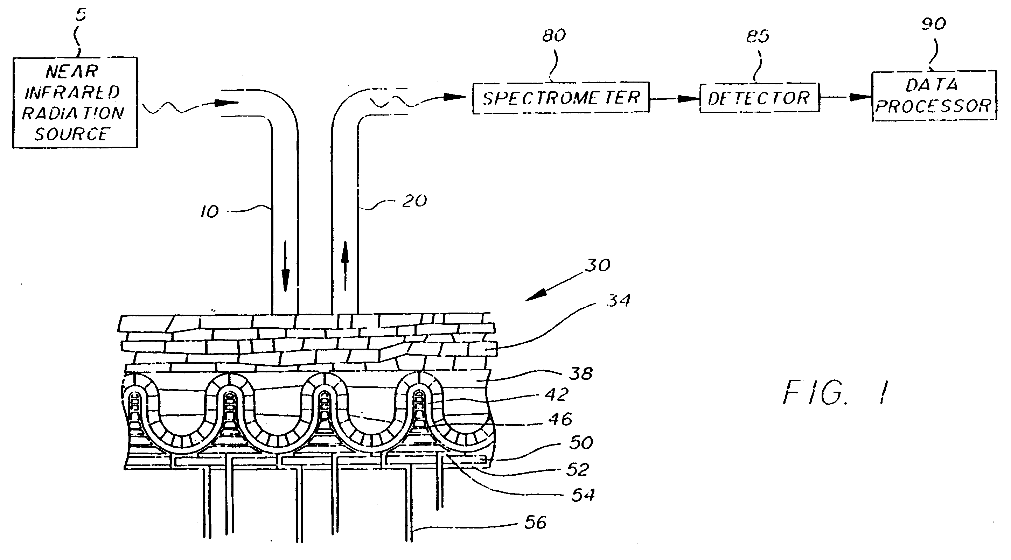

Referring to Figure 1, there is shown, in section, a schematic representation of the skin of an individual during the non-invasive determination of glucose concentration in blood. Incident infrared radiation is transmitted by incident optical fiber 10 into skin 30 of the individual. Back-scattered infrared radiation from skin 30 is received in pickup optical fiber 20 and transmitted to glucose concentration sensor 70. Incident optical fiber 10 and pick-up optical fiber 20 may each be a bundle of optical fibers. Optical fiber bundle 10 and optical fiber bundle 20 may be in any desired geometric relationship. Within the skin, radiation travels through a dead cell layer 34, a layer of live cells, or basal layer, 38, before entering the capillary region, and specifically papillae 42. The papillae each contain a large numbers of capillaries 46 and live cells.

If radiation penetrates still deeper in skin 30 before being back-scattered, it will enter the portion of the dermis containing the venules 52 and arterioles of the superficial plexus 50.

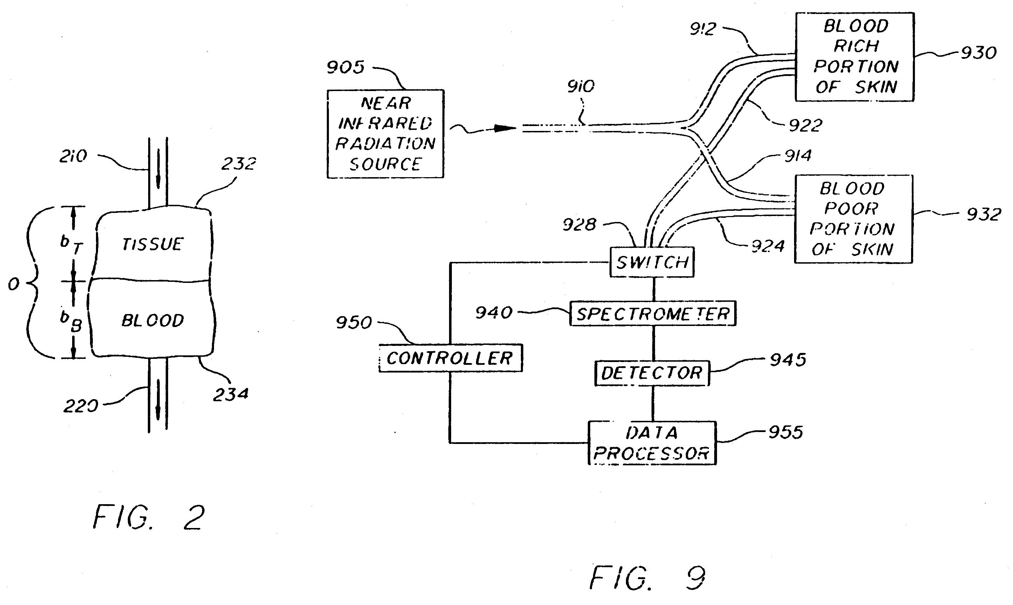

Referring to Figure 2, there is shown schematically the transmission of radiation through blood and tissue. Radiation is transmitted from an emitter (not shown) through first optical fiber 210 into skin 230. Radiation from optical fiber 210 passes through a length $b_T$ of tissue 232 and a length $b_B$ of blood 234 before being received in pickup optical fiber 220 and transmitted to a detector (not shown). According to Beers' Law, the intensity of transmitted or transflected radiation $I_T$, is given by:

$$I_T = I_o \times 10^{-A_G}$$

where $I_0$ is the intensity of the incident radiation, and $A_G$ is the absorbance.

The absorbance $A_G$ of radiation by glucose in the example shown schematically in Figure 2 may be represented as

$A_G = a_G\, b_B\, C_{GB} + a_G\, b_T\, C_{GT}$

where $C_{GB}$ is the concentration of glucose in blood, $C_{GT}$ is the concentration of glucose in tissue, and $a_G$ is a constant characteristic of the absorbance of light by glucose.

Multivariate statistical techniques, such as partial least squares, can be used to determine $K_1 = b_B\, a_G$ and $K_2 = b_T\, a_G$, but cannot be employed to solve the equation for the absorbance due to glucose, $A_G = K_1\, C_{GB} + K_2\, C_{GT}$, which is a single equation in two unknowns.

None of the existing techniques for infrared non-invasive detection of blood glucose concentrations has attempted to account for the contribution to the total absorption by glucose in tissue. There are only two circumstances under which accurate measurements of blood glucose concentrations could be obtained, without allowing for the contribution by glucose in tissue. If there is no glucose in the cells, then all glucose detected by the instrument will be glucose contained in the blood. If the concentration of glucose in the cells is equal to that in the bloodstream, then the combined absorption of the glucose in the bloodstream and the glucose in the cells will provide results that will permit detection of the concentration in the bloodstream.

In fact, neither of the above two scenarios is correct. The concentration of glucose in living cells is not negligible. Moreover, in living cells, glucose is metabolized into glucose phosphate. The absorption spectrum of glucose phosphate is very similar to that of glucose. Accordingly, glucose phosphate will, by instruments using present technology, be detected as glucose.

The concentration of glucose in the bloodstream and in the cells is not identical. In particular, in persons suffering from diabetes, who are most likely to employ non-invasive glucose sensing techniques, it has been observed that there is little predictable relation between the concentration of glucose in the blood and the concentration of glucose in the tissues.

As a result, it is not possible accurately to calibrate a non-invasive glucose sensor of the prior art.

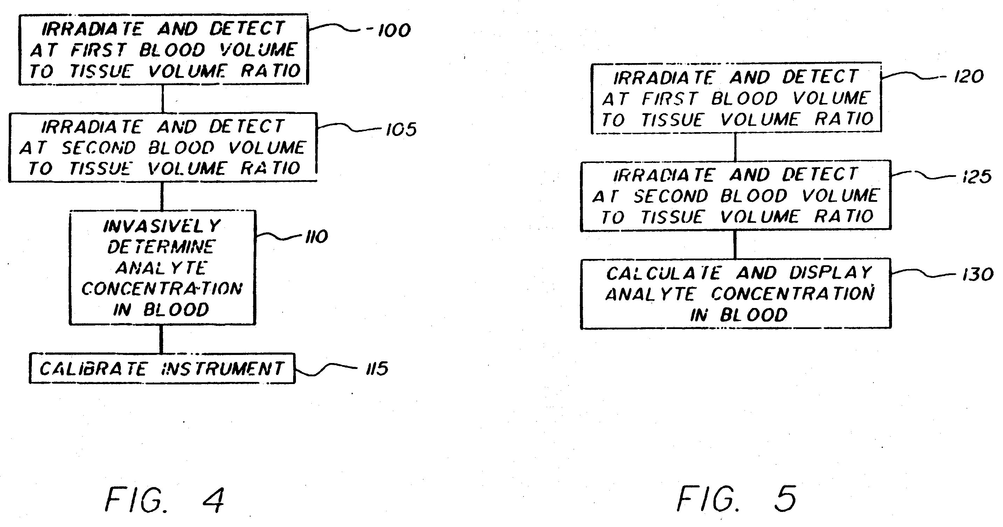

In a calibration technique for an instrument for non-invasively determining the concentration of an analyte in the blood of a living animal according to the invention, a body part of an animal having a first blood volume to tissue volume ratio is irradiated, and reflected or absorbed radiation emitted from the body part is detected, as is conventional. Referring to Figure 4, this step is illustrated in box 100 as "IRRADIATE AND DETECT AT FIRST BLOOD VOLUME TO TISSUE VOLUME RATIO." This step may be accomplished using, as shown in Figure 1, conventional techniques. Infrared radiation source 5 may be a tungsten filament bulb, maintained thermally isolated, with a constant current through the filament and an infrared filter intermediate the bulb and optical fiber bundle 10. Radiation source 5 preferably provides radiation in a continuous spectrum between about 1000nm and about 2500nm. Pick-up optical fiber (or bundle of optical fibers) 20 transmits radiation emitted from the body part 30 to spectrometer 80. For example, in Figure 1, radiation emitted from skin 30 transmitted by pickup optical fiber 20 to spectrometer 80, which spectrally separates the radiation, and focuses the radiation on detector 85. Spectrometer 80 may be, for example, a unitary block of appropriate glass in a Czerny-Turner configuration. Detector 85 may be, as is conventional in infrared and near-infrared detection, an array of lead-sulfide detectors. A selected portion of the spectrum is focused by the spectrometer 80 on detector 85. For example, detector 85 may include 64 detectors, each covering a wavelength range of about 15nm. Electrical signals from detector 85 are transmitted to data processor 90 which, in accordance with conventional data processing techniques, obtains a first absorbance spectrum, showing absorbance plotted against wavelength and stores that first absorbance spectrum. A pre-amplifier, an amplifier and an analog-to-digital converter may be provided intermediate the detector array and data processor 90. A chopper is also preferably provided in the path of reflected light to modulate the radiation and, in combination with a lock-in amplifier, reduce the effects of noise.

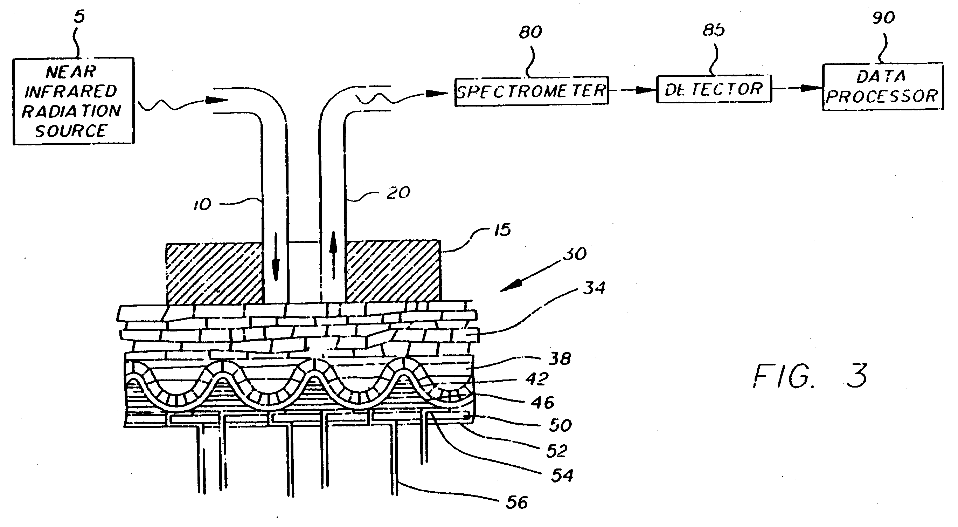

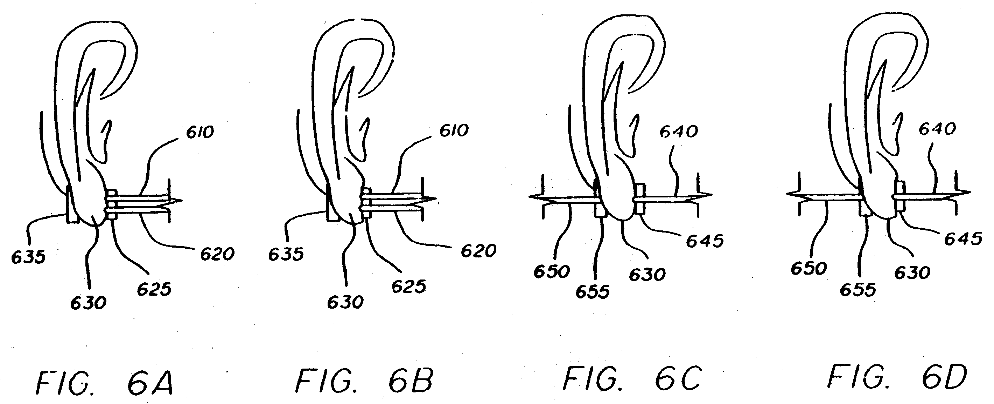

Referring again to Figure 4, the next step, indicated at block 105, is "IRRADIATE AND DETECT AT SECOND BLOOD VOLUME TO TISSUE VOLUME RATIO." Carrying out of this step is illustrated in Figure 3. It will be seen, referring to Figure 3, that a mass 15, disposed about optical fibers 10 and 20, is compressing skin 30. Compression of skin 30 does not affect substantially the volume of dead cells 34, cells in basal layer 38, or living cells in papillae 42 or deeper layers of the dermis. However, it will be observed that capillaries 46 in the papillae are significantly compressed. As a result, the ratio of blood volume to tissue volume is decreased. Thus a second blood volume to tissue volume ratio is obtained. Alternative techniques for carrying out of this step are explained below with reference to Figures 6-11. Upon irradiation and emission of radiation, emitted radiation is transmitted through pick-up fiber (or bundle of fibers) 20 to spectrometer 80, and detected by detector 85, which provides an electrical signal to data processor 90. Data processor 90 calculates an absorbance spectrum for irradiation at the second blood volume to tissue volume ratio and stores that absorbance spectrum.

The next step, as shown in Figure 4, at block 110, is to "INVASIVELY DETERMINE ANALYTE CONCENTRATION IN BLOOD." This step is performed, in accordance with conventional techniques, by lancing a body part, such as a finger, to obtain a small quantity of blood, and then analyzing the blood in a high accuracy instrument. For example, in order to obtain the concentration of glucose invasively, an analyzer manufactured by Yellow Spring Instruments may be employed.

The next step is the calibration of the instrument, as shown by block 115 in Figure 4, labeled "CALIBRATE INSTRUMENT." The calibration step is carried out, preferably using multi-variate analytical techniques, using as data inputs the absorbance spectrum obtained at the first blood volume to tissue volume ratio, the absorbance spectrum obtained at the second blood volume to tissue volume ratio and the analyte concentration determined from analysis of the invasively-obtained blood sample. In a preferred embodiment, the multivariate analytical technique is the method of partial least squares. Various commercial software packages are available that will perform the computations required for partial least squares analysis. Such software packages include, for example, NSAS, by NIR Systems of Silver Spring, Maryland, and Spectra Calc, Lab-Calc and GRAMS, by Galactor Industries, of Salem, New Hampshire. Those of skill in the art in performing partial least squares analysis will be able to input properly the first blood volume to tissue volume ratio absorbance spectrum, the second blood volume to tissue volume ratio absorbance spectrum, and the analyte concentration determined from the invasively-obtained blood sample, in order to obtain a set of factors. The set of factors will, when multiplied by a given spectrum, provide the concentration of the desired analyte in the blood.

Referring now to Figure 5, the steps in non-invasively obtaining an analyte concentration, such as the concentration of glucose, in the blood of a living animal according to a process of the invention will be explained. The first step, as shown in block 120 of Figure 5, is to "IRRADIATE AND DETECT AT FIRST BLOOD VOLUME TO TISSUE VOLUME RATIO." This process is discussed above and illustrated in Figure 1. Data processor 90 stores an absorbance spectrum for the first blood volume to tissue volume ratio. The next step, as illustrated by block 125 of Figure 5, is to "IRRADIATE AND DETECT AT SECOND BLOOD VOLUME TO TISSUE VOLUME RATIO." This step may be accomplished while compressing the skin, as illustrated in Figure 3, or by one of the techniques explained below in connection with Figures 6-11. The emitted radiation is then received by detector 85, as illustrated in Figure 1. Detector 85 provides an output electrical signal representing the spectrum to data processor 90, which calculates and stores a absorption spectrum for the second blood volume to tissue volume ratio. Data processor 90 will then, in accordance with conventional techniques, calculate the concentration of the analyte in blood, using a set of factors calculated during calibration of the instrument, as discussed above in connection with Figure 4. This concentration is then preferably displayed on a suitable display. The reading may also be stored in an appropriate memory device. This step of calculation and display is indicated in Figure 5, at block 130, labeled "CAL-CULATE AND DISPLAY ANALYTE CONCENTRA-TION IN BLOOD."

There will now be explained and illustrated a number of specific techniques for obtaining readings at a first blood volume to tissue volume ratio and a second blood volume to tissue volume ratio. Referring to Figures 6A and 6B, there is illustrated a technique for obtaining readings from the ear lobe of a human patient. Input optical fiber (which may also be a bundle of optical fibers) 610 is in contact with the skin of ear lobe 630. Pickup optical fiber (which may also be a bundle of optical fibers) 620 is also in contact with the skin of ear lobe 630. There is disposed about optical fibers 610, 620 a rigid ring 625. There is provided on the opposite side of earlobe 630 a mass 635. As shown in Figure 6A, an initial reading, at a first blood volume to tissue volume ratio, is obtained with no pressure on the earlobe. In accordance with conventional techniques, an appropriate instrument (not shown) obtains an absorbance spectrum for this reading. Referring to Figure 6B, ring 625 and mass 635 compress ear lobe 630. As a result, the quantity of blood in ear lobe 630 will be substantially less than the blood volume when ear lobe 630 is not compressed as in Figure 6A. Thus, a second blood volume to tissue volume ratio is obtained. A reading is then taken at this second blood volume to tissue volume ratio, and an absorbance spectrum determined. These readings may either be used in a calibration process, or, if an instrument has already been calibrated, in determining the glucose or other blood analyte level, non-invasively.

Referring now to Figures 6C and 6D, there is illustrated a technique for transmitting radiation through the earlobe of a human patient to obtain an absorbance reading. Input optical fiber (which may also be a bundle of optical fibers) 640, is in contact with the skin of earlobe 630, at one side thereof. Pickup optical fiber (which may also be a bundle of optical fibers) 650 is also in contact with the skin of earlobe 630, but opposite to input optical fiber 640. There is disposed about input optical fiber 640 a rigid block 645. There is provided about pickup optical fiber 650 a rigid block 655. As shown in Figure 6C, an initial reading, at a first blood volume to tissue volume ratio, is obtained with no pressure on the earlobe. In accordance with conventional techniques, an appropriate instrument (not shown) obtains an absorbance spectrum for this reading. Referring to Figure 6B, earlobe 630 is compressed between masses 645 and 655. As a result, the quantity of blood in earlobe 630 will be substantially less than the blood volume when earlobe 630 is not compressed as in Figure 6C. Thus, a second blood volume to tissue volume ratio is obtained. A reading is then taken at this second blood volume to tissue volume ratio, and an absorbance spectrum determined. These readings may either be used in a calibration process, or, if an instrument has already been calibrated, in determining the glucose or other blood analyte level, non-invasively.

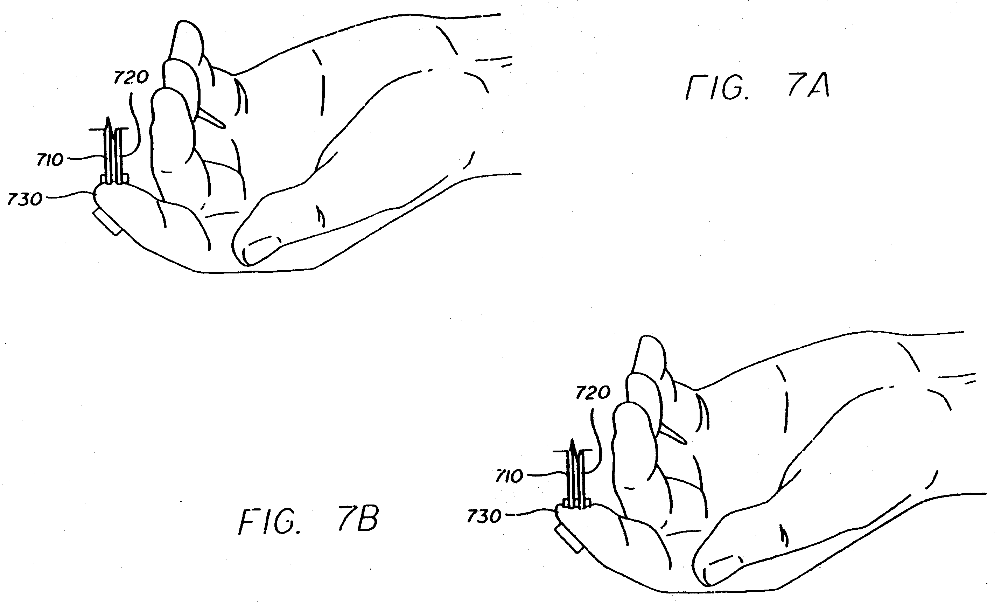

Referring now to Figures 7A and 7B, there will be explained a method of non-invasively calibrating an instrument, and detecting a blood analyte concentration, by taking readings at a patient's finger. Referring to Figure 7A, there is shown a person's finger tip 730 with incident optical fiber or optical fiber bundle 710, and pick up optical fiber or optical fiber bundle 720, in contact with the finger and adjacent to one another. There is in finger tip 730 a first blood volume to tissue volume ratio. Incident optical fiber 710 transmits infrared radiation into finger tip 730. Radiation emitted from finger 730 into pick up optical fiber 720 is transmitted to an analysis instrument, which obtains an absorbance spectrum for a first blood volume to tissue volume ratio. Referring now to Figure 7B, finger tip 730 has been compressed between ring 735 and surface 740. Compression of finger tip 730 results in a lower, second, blood volume to tissue volume ratio. Radiation transmitted through incident optical fiber 710 enters finger tip 730. A portion of that radiation is emitted into pickup optical fiber 720 and transmitted to an analysis instrument, which calculates an absorbance spectrum for the second blood volume to tissue volume ratio. If the technique of Figures 7A and 7B is employed in calibrating an analysis instrument, a blood sample will be obtained, and concentration of the selected analyte measured by conventional analytical techniques. Using the data from the two absorbance spectra and the invasive measurement, the instrument will be calibrated. Alternatively, if the instrument is already calibrated, the instrument will, from the two absorption spectra, calculate the concentration of the blood analyte of interest, and display and/or store that concentration.

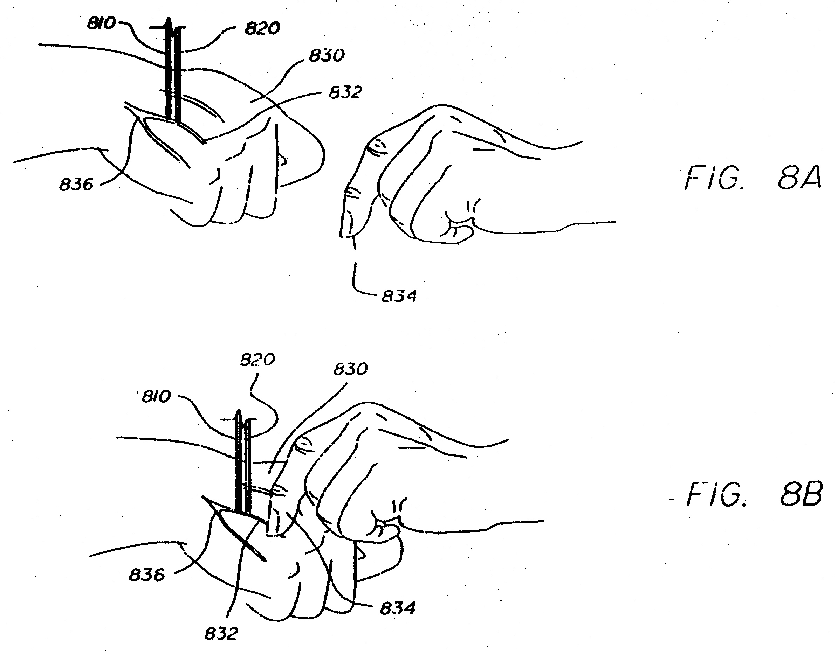

Referring to Figures 8A and 8B, there is illustrated a method according to the invention employing readings of large veins close to the skin surface. In Figure 8A, there is shown a body part, such as the hand 830 of an individual, with a prominent vein 832 located close to the skin. An incident optical fiber or optical fiber bundle 810 transmits radiation from an emitter (not shown) to the body part 830. Radiation emitted from body part 830 into pickup optical fiber or pick up optical fiber bundle 820 is transmitted to a conventional instrument for obtaining of an absorbance spectrum. A reading is taken at a first blood volume to tissue volume ratio, as illustrated in Figure 8A, to obtain a first absorbance spectrum. Because the large vein is close to the surface, the blood volume to tissue volume ratio is relatively high. In Figure 8B, a reading is taken at a second, lower, blood volume to tissue volume ratio. In Figure 8B, finger 834 has been used to drain vein 832 of blood. This is done simply by pressing the finger on the vein, and running the finger outward from the heart while maintaining pressure on the vein. After the finger passes over a valve in the vein, blood will not flow outward beyond the valve to refill the vein. The location of a valve is indicated at 836 in Figure 8B. Consequently, there is a portion of the vein, intermediate finger 834 and the valve at 836, which will be substantially empty of blood. A second reading is taken, and a second absorbance spectrum is obtained at this second, lower, blood volume to tissue volume ratio. As appropriate, either the instrument is then calibrated, or the blood glucose or other analyte concentration is calculated and displayed, as explained above.

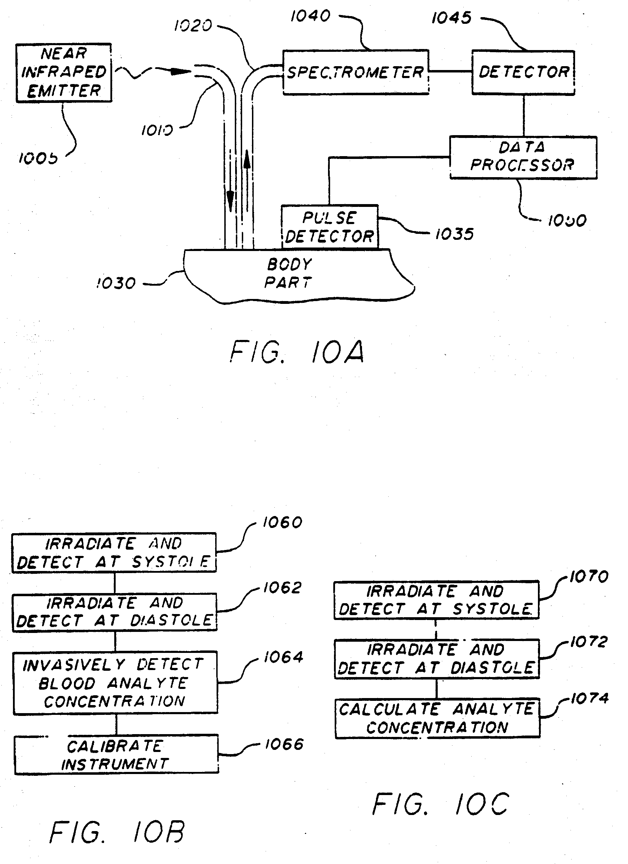

Referring now to Figure 9, there is schematically illustrated an apparatus for accomplishing a method of the invention in an embodiment in which the differences in blood volume to tissue volume ratio in different portions of the skin are employed to obtain two different readings. Near-infrared radiation source 905 emits radiation in the near-infrared into an incident optical fiber or incident optical fiber bundle 910. Incident optical fiber bundle 910 splits into first branch incident optical fiber bundle 912 and second branch incident optical fiber bundle 914. First branch incident optical fiber bundle 912 is disposed with its end in contact with a blood rich portion of skin 930 of a patient. Blood rich portion of the skin 930 may be, for example, the inside of the wrist. Any other portion of the skin where a substantial number of blood vessels are located close to the surface, would be a suitable blood rich portion of the skin. The end of second branch incident optical fiber bundle 914 is placed in contact with a blood poor portion of skin 932. Blood poor portion 932 may be, for example, the upper interior forearm.

Infrared radiation emitted from blood rich portion of skin 930 enters pickup optical fiber or pickup optical fiber bundle 922 and is transmitted through optical fiber bundle 922 to optical switch 928. Infrared radiation from blood poor portion of skin 932 is received by pickup optical fiber or pickup optical fiber bundle 924 and is transmitted through optical fiber 924 to optical switch 928. Optical switch 928 selectively allows radiation from either pickup optical fiber bundle 922 or pickup optical bundle 924 to enter spectrometer 940. Spectrometer 940 spectrally separates radiation received from pickup optical fiber bundle 922 or 924, and focuses radiation on optical detector 945. Optical detector 945 provides an electrical signal representative of detected radiation to data processor 955. Controller 950 controls the operation of optical switch 928, and provides signals to data processor 955, so that data processor 955 can accurately distinguish the spectra from blood rich portion of skin 930 and blood poor portion of skin 932. Data processor 955 calculates an absorbance spectrum for blood rich portion of skin 930 and blood poor portion of skin 932. These absorbance spectra are processed, as explained above, either to calibrate an instrument or to calculate analyte concentration in the blood.

Referring now to Figure 10A, there is shown schematically an apparatus for obtaining an absorbance spectrum at two different blood volume to tissue volume ratios in accordance with the method of the invention. Near infrared emitter 1005 emits radiation in the near infrared. This near infrared radiation is transmitted into incident optical fiber bundle 1010. An end of incident optical fiber bundle 1010 is placed in contact with the skin of body part 1030, so that infrared radiation irradiates body part 1030. Radiation emitted from body part 1030 is received in pickup optical fiber or optical fiber bundle 1020 and is transmitted to spectrometer 1040. Radiation from body part 1030 is then spectrally separated and focused on detector array 1045. Detector 1045 provides an electrical signal to data processor 1050 indicative of the light intensity. Data processor 1050 calculates an absorption spectrum.

Simultaneously, pulse detector 1035 is physically coupled to the skin of body part 1030. Pulse detector 1035 is electrically coupled to, and provides an electrical signal to, data processor 1050, which signal indicates the pulse cycle in body part 1030. Pulse detector 1035 may be, for example, a pressure sensor or an optical sensor that detects hemoglobin variance. As a result, pulse detector 1035 provides information regarding the relative quantity of blood in blood vessels in body part 1030. During the cycle of the pulse in any body part, the blood volume varies and thus the blood volume to tissue volume ratio varies.

Referring now to Figure 10B, there will be explained a method for calibrating an instrument using the device of Figure 10A in a method according to the invention. In the first step, illustrated at block 1060, labeled "IRRADIATE AND DETECT AT SYSTOLIC," the step of irradiating body part 1030 during the systolic portion of the pulse cycle, as detected by pulse detector 1035, is indicated. In the next step, body part 1030 is irradiated during the diastolic portion of the pulse cycle, as detected by pulse detector 1035. This step is illustrated by clock 1062, labeled "IRRADIATE AND DETECT AT DIASTOLIC." Data processor 1050 has then calculated and stored absorbance spectra obtained at the systolic and diastolic portions of the pulse cycle. The absorbance spectrum obtained at the systolic portion of the cycle represents a relatively high blood volume to tissue volume ratio. The absorbance obtained in the diastolic portion of the cycle represents an absorbance spectrum obtained at a relatively low blood volume to tissue volume ratio.

The next step in the method of calibration according to Figure 10B is to obtain a blood sample and to detect the concentration of the blood analyte of interest from this sample using conventional techniques, as explained in greater detail above. This step is illustrated at block 1064, labeled "INVASIVELY DETECT BLOOD ANALYTE CONCENTRATION." The next step is to calibrate the instrument, preferably in accordance with multivariate techniques, such as partial least squares. This step is illustrated at block 1066, labeled "CALIBRATE INSTRUMENT."

Referring now to Figure 10C, there are illustrated the steps in non-invasively measuring a blood analyte concentration using the apparatus illustrated in Figure 10A. The first step is to irradiate body part 1030 and detect the emitted radiation during the systolic portion of the pulse cycle. The portion of the pulse cycle is, as explained above, detected by pulse detector 1035. This first step is illustrated at block 1070, labeled "IRRADIATE AND DETECT AT SYSTOLIC." The next step is to irradiate body part 1030 at the diastolic portion of the pulse cycle. The portion of the pulse cycle is detected by pulse detector 1035, which provides an appropriate electrical signal to data processor 1050. This step is illustrated by block 1072, labeled "IRRADIATE AND DETECT AT DIASTOLIC." Data processor 1050 has now obtained and calculated absorbance spectra for a first blood volume to tissue volume ratio and a second blood volume to tissue volume ratio. As calibration has already been performed, the blood analyte concentration may now be calculated. This step of calculation is illustrated at block 1074 labeled "CALCULATE BLOOD ANALYTE CONCENTRATION."

It will be understood that in fact near infrared emitter 1005 may, through incident optical fiber bundle 1010, irradiate body part 1030 continuously over several pulse cycles. The signal produced by pulse detector 1035 will then be used by data processor 1050 to characterize data received from detector 1045 as to the portion of the pulse cycle to which such data relates. In addition, it will be understood that readings may be taken initially at any portion of the cycle, and not merely at the systolic portion of the cycle, as illustrated in Figures 10B and 10C.

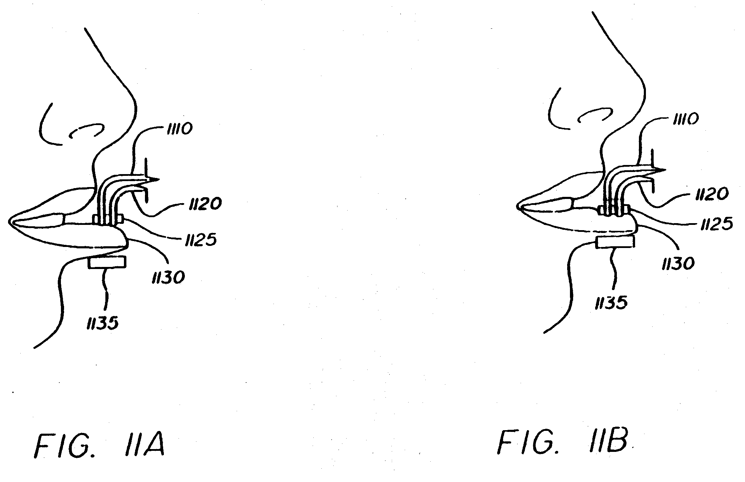

Referring now to Figure 11A and 11B there is illustrated a technique for obtaining readings from the interior of the mouth of a human patient. The technique illustrated in Figures 11A and 11B is performed on the interior of the lip, and in particular, the interior of the lower lip, of the patient. The same technique may be applied to other interior surfaces of the mouth. Input optical fiber (which may also be a bundle of optical fibers) 1110 is in contact with the interior surface of lower lip 1130. Pickup optical fiber (which may also be a bundle of optical fibers) 1120 is also in contact with the interior surface of lower lip 1130. There is disposed about optical fibers 1110, 1120 a rigid ring 1125. There is provided on the outer surface of lip 1130, opposite to rigid ring 1125, a mass 1135. As shown in Figure 11A, an initial reading, at a first blood volume to tissue volume ratio, is obtained with no pressure on the lip. In accordance with conventional techniques, an appropriate instrument (not shown) obtains an absorbance spectrum for this reading. Referring to Figure 11B, ring 1125 and mass 1135 compress lip 1130. As a result, the quantity of blood in lip 1130 intermediate rigid ring 1125 and mass 1135 will be substantially less than the blood volume in that portion of the lip when lip 1130 is not compressed as in Figure 11A. Thus, a second blood volume to tissue volume ratio is obtained. A reading is taken at this second blood volume to tissue volume ratio, and an absorbance spectrum determined. These readings, as with the examples above, may either be used in a calibration process, or, if an instrument has already been calibrated, in determining the glucose or other blood analyte level, non-invasively.

It will be understood that other techniques may be employed to obtain absorbance spectra at a first blood volume to tissue volume ratio and a second blood volume to tissue volume ratio. The various techniques set forth in connection with Figures 6 through 11 are merely examples of such techniques.

It will be appreciated that there are considerable variations that can be accomplished in a method and apparatus of the invention without departing from its scope. As a result, although a preferred embodiment of a method and apparatus of the invention has been described above, it is emphasized that the invention is not limited to a preferred embodiment and there exist other alternative embodiments that are fully encompassed within the invention's scope, which is intended to be limited only by the scope of the appended claims.

## Claims

1. A method for the detection of the concentration of a constituent of blood of a living animal or person, comprising the steps of:

    (a) non-invasively irradiating a body part having a first blood volume to tissue volume ratio and detecting radiation emitted from the body part;

    (b) non-invasively irradiating a body part having a second blood volume to tissue volume ratio and detecting radiation emitted from the body part; and

    (c) calculating the concentration of the constituent in blood based on the radiation detected in said steps (a) and (b).

2. The method of claim 1, wherein the body part having a first blood volume to tissue volume ratio and the body part having a second blood volume to tissue volume ratio are the same body part.

3. The method of claim 2, wherein, simultaneously with said step (b), pressure is applied to the body part to decrease the blood volume to tissue volume ratio in the body part.

4. The method of claim 3, wherein the body part is the ear lobe.

5. The method of claim 4, wherein the emitted detected radiation has been transmitted through the earlobe.

6. The method of claim 3, wherein the body part is the finger.

7. The method of claim 3, wherein the body part is the interior of the mouth.

8. The method of claim 2, wherein said step (a) is performed during a first portion of the pulse cycle of the animal or person, and wherein said step (b) is performed at a second portion of the pulse cycle of the animal.

9. The method of claim 2, wherein the body part includes a vein close to the skin, and, in one of said steps (a) and (b), the vein has been manually drained of substantially all blood by non-invasively compressing the vein.

10. The method of claim 1, wherein said step (a) and said step (b) are performed simultaneously, and wherein the body part having a first blood volume to tissue volume ratio and the body part having a second blood volume to tissue volume ratio, are different body parts.

11. A method for calibrating an instrument for non-invasively determining the concentration of an analyte in the blood of a living animal or person, comprising the steps of:
   (a) non-invasively irradiating a body part of the animal having a first blood volume to tissue volume ratio and detecting radiation emitted from the body part to obtain a first absorbance spectrum;
   (b) non-invasively irradiating a body part of the animal having a second blood volume to tissue volume ratio and detecting radiating emitted by the body part to obtain a second absorbance spectrum;
   (c) invasively detecting the concentration of the analyte in the blood of the animal substantially simultaneously with said steps (a) and (b); and
   (d) employing the first absorbance spectrum, the second absorbance spectrum, and the invasively-detected concentration, to calibrate the instrument.

12. The method of claim 11, wherein said step (d) comprises employing a multivariate analytical technique.

13. The method of claim 12, wherein said multivariate analytical technique is partial least squares.

14. The method of claim 11, wherein the body part having a first blood volume to tissue volume ratio and the body part having a second blood volume to tissue volume ratio are the same body part.

15. The method of claim 14, wherein, simultaneously with said step (b), pressure is applied to the body part to decrease the blood volume to tissue volume ratio in the body part.

16. The method of claim 15, wherein the body part is the ear lobe.

17. The method of claim 16, wherein the emitted detected radiation has been transmitted through the earlobe.

18. The method of claim 15, wherein the body part is the finger.

19. The method of claim 13, wherein the body part is the interior of the mouth.

20. The method of claim 14, wherein said step (a) is performed during a first portion of the pulse cycle of the animal or person, and wherein said step (b) is performed during a second portion of the pulse cycle of the animal.

21. The method of claim 14, wherein the body part includes a vein close to the skin, and, in one of said steps (a) and (b), the vein has been manually drained of substantially of all blood by non-invasively compressing the vein.

22. The method of claim 11, wherein said step (a) and said step (b) are performed simultaneously, and wherein the body part having a first blood volume to tissue volume ratio and the body part having a second blood volume to tissue volume ratio, are different body parts.

23. An apparatus for the non-invasive detection of the concentration of a constituent of blood of a living animal or person, comprising:
   (a) means (10) for non-invasively irradiating a body part (20) of the animal or person,
   (b) means (20) for detecting radiation emitted from the body part; and
   (c) data processing means (80,85,90) for receiving a first signal from said detecting means (20) representative of the intensity of radiation detected during irradiation of a body part having a first blood volume to tissue volume ratio and calculating and storing a first absorbance spectrum based on said first signal; receiving a second signal from said detecting means (20) representative of the intensity of radiation detected during irradiation of a body part having a second blood volume to tissue volume ratio and calculating and storing a second absorbance spectrum based on said second signal; and calculating from said first absorbance spectrum and said second absorbance spectrum the concentration of the constituent in the blood.

24. An apparatus for calibrating an instrument for non-invasive determination of the concentration of an analyte in the blood of a living animal or person, comprising:

(a) means (10) for non-invasively irradiating a body part of the animal or person;

(b) means (20) for detecting radiation emitted from the body part; and

(c) data processing means (80,85,90) for receiving a first signal from said detecting means (20) representative of the intensity of radiation detected during irradiation of a body part having a first blood volume to tissue volume ratio and calculating and storing a first absorbance spectrum based on said first signal; receiving a second signal from said detecting means (20) representative of the intensity of radiation detected during irradiation of a body part having a second blood volume to tissue volume ratio and calculating and storing a second absorbance spectrum based on said second signal; receiving an input representing an invasively-detected concentration of the analyte in the blood of the animal; and based on said first absorbance spectrum, said second absorbance spectrum, and said invasively-detected concentration, calibrating the instrument.

NEAR INFRARED RADIATION SOURCE

SPECTROMETER

DETECTOR

DATA PROCESSOR

FIG. 1

FIG. 2

FIG. 9

EP 0 623 308 A1

NEAR INFRARED RADIATION SOURCE — 5

10   20   15

30   34   38   42   46   50   52   54   56

SPECTROMETER — 80

DETECTOR — 85

DATA PROCESSOR — 90

FIG. 3

EP 0 623 308 A1

```
┌─────────────────────────┐
│   IRRADIATE AND DETECT   │─── -100
│   AT FIRST BLOOD VOLUME  │
│   TO TISSUE VOLUME RATIO │
└─────────────────────────┘
             │
┌─────────────────────────┐
│   IRRADIATE AND DETECT   │─── 105
│  AT SECOND BLOOD VOLUME  │
│   TO TISSUE VOLUME RATIO │
└─────────────────────────┘
             │
┌─────────────────────────┐
│        INVASIVELY        │─── 110
│        DETERMINE         │
│         ANALYTE          │
│      CONCENTRATION       │
│         IN BLOOD         │
└─────────────────────────┘
             │
┌─────────────────────────┐
│  CALIBRATE INSTRUMENT    │─── 115
└─────────────────────────┘
```

FIG. 4

```
┌─────────────────────────┐
│   IRRADIATE AND DETECT   │─── 120
│   AT FIRST BLOOD VOLUME  │
│   TO TISSUE VOLUME RATIO │
└─────────────────────────┘
             │
┌─────────────────────────┐
│   IRRADIATE AND DETECT   │─── 125
│  AT SECOND BLOOD VOLUME  │
│   TO TISSUE VOLUME RATIO │
└─────────────────────────┘
             │
┌─────────────────────────┐
│   CALCULATE AND DISPLAY  │─── 130
│  ANALYTE CONCENTRATION   │
│         IN BLOOD         │
└─────────────────────────┘
```

FIG. 5

EP 0 623 308 A1

FIG. 6A    FIG. 6B    FIG. 6C    FIG. 6D

EP 0 623 308 A1

FIG. 7A

FIG. 7B

720
710
730

EP 0 623 308 A1

FIG. 8A

FIG. 8B

NEAR INFRARED EMITTER — 1005

1020

1010

SPECTROMETER — 1040

DETECTOR — 1045

DATA PROCESSOR — 1050

PULSE DETECTOR — 1035

BODY PART — 1030

FIG. 10A

IRRADIATE AND DETECT AT SYSTOLE — 1060

IRRADIATE AND DETECT AT DIASTOLE — 1062

INVASIVELY DETECT BLOOD ANALYTE CONCENTRATION — 1064

CALIBRATE INSTRUMENT — 1066

FIG. 10B

IRRADIATE AND DETECT AT SYSTOLE — 1070

IRRADIATE AND DETECT AT DIASTOLE — 1072

CALCULATE ANALYTE CONCENTRATION — 1074

FIG. 10C

*FIG. IIB.*

*FIG. IIA*

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X X X | US-A-5 111 817 (J.S. CLARK ET AL.) <br> * column 4, line 15 - column 6, line 27 * <br> * column 10, line 18 - column 12, line 4 * | 1-6,8,11 14-18,20 23,24 | A61B5/00 |
| X X X | US-A-4 714 341 (K. HAMAGURI ET AL.) <br> * column 5, line 24 - line 61 * <br> * column 27, line 12 - column 28, line 35 * | 1-6,8,11 14-18,20 23,24 | |
| X <br><br> X X | US-A-4 927 264 (T. SHIGA ET AL.) <br><br> * column 2, line 47 - column 3, line 3 * <br> * column 4, line 26 - column 6, line 37 * | 1-3,6,8, 9,11 15,18,20 21,23,24 | |
| X X X | EP-A-0 444 934 (HEWLETT-PACKARD CO.) <br> * column 3, line 37 - line 57 * <br> * column 5, line 23 - column 6, line 9 * | 1,3,6,8 10,11,15 18,22-24 | |
| X X | EP-A-0 290 278 (HAMAMATSU PHOTONICS K.K.) <br> * page 4, line 51 - page 5, line 42 * | 1,10,11 22-24 | |
| | | | TECHNICAL FIELDS SEARCHED (Int.Cl.5) <br><br> A61B |

The present search report has been drawn up for all claims

| Place of search | Date of completion of the search | Examiner |
|---|---|---|
| THE HAGUE | 12 August 1994 | Rieb, K.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)